# EUROPÄISCHE PATENTANMELDUNG

(11) **EP 1 159 926 A2**
(43) Veröffentlichungstag der Anmeldung: **05.12.2001**
(21) Anmeldenummer: 01112303.1
(22) Anmeldetag: 19.05.2001
(51) Int. Cl.: A61B 18/14, A61B 17/32

(54) **Scheren- oder zangenförmiges chirurgisches Instrument**

(30) Priorität: 03.06.2000 DE 10027727
(71) Anmelder: AESCULAP AG, 78532 Tuttlingen (DE)
(72) Erfinder: Dworschak, Manfred, 78589 Dürbheim (DE); Frei, Gerhard, 88637 Buchheim (DE); Mayenberger, Rupert, 78239 Rielasingen (DE); Rothweiler, Christoph, 78166 Donaueschingen (DE); Weisshaupt, Dieter, 78194 Immendingen (DE)
(74) Vertreter: Hoeger, Stellrecht & Partner

(57) **Zusammenfassung**

Um bei einem scheren- oder zangenförmigen chirurgischen Instrument mit zwei relativ zueinander beweglich miteinander verbundenen Branchen, die jeweils ein Werkzeug tragen, wobei die Branchen und die Werkzeuge elektrisch voneinander isoliert sind und jedes Werkzeug über eine Branche mit jeweils einem Pol einer externen Spannungsquelle in Verbindung steht, die Handhabbarkeit im Anschlußbereich des Instrumentes zu verbessern, wird vorgeschlagen, daß zu einer ersten Branche ein zweiadriges, zu den Polen der externen Spannungsquelle führendes Versorgungskabel führt, dessen erste Ader direkt mit dieser ersten Branche verbunden ist, während dessen zweite Ader über ein Verbindungskabel unter Ausbildung einer von der ersten Branche zur zweiten Branche führenden Schlaufe mit der zweiten Branche verbunden ist.

## Beschreibung

Die Erfindung betrifft ein scheren- oder zangenförmiges chirurgisches Instrument mit zwei relativ zueinander beweglich miteinander verbundenen Branchen, die jeweils ein Werkzeug tragen, wobei die Branchen und die Werkzeuge elektrisch voneinander isoliert sind und jedes Werkzeug über eine Branche mit jeweils einem Pol einer externen Spannungsquelle in Verbindung steht.

Ein derartiges Instrument ist beispielsweise aus der DE 198 28 976 A1 bekannt. Bei diesem Instrument ist mit jeder Branche ein eigenes Anschlußkabel verbunden, die Anschlußkabel laufen getrennt zu einer Spannungsquelle, beispielsweise einem Hochfrequenzgenerator, so daß bei der Handhabung die Gefahr besteht, daß sich die Anschlußkabel verdrillen oder verknoten.

Ausgehend von diesem Stand der Technik liegt der Erfindung die Aufgabe zugrunde, die Handhabung eines gattungsgemäßen Instrumentes insbesondere im Bereich der elektrischen Anschlüsse zu verbessern.

Diese Aufgabe wird bei einem Instrument der eingangs beschriebenen Art erfindungsgemäß dadurch gelöst, daß zu einer ersten Branche ein zweiadriges, zu den Polen der externen Spannungsquelle führendes Versorgungskabel führt, dessen erste Ader direkt mit dieser ersten Branche verbunden ist, während dessen zweite Ader über ein Verbindungskabel unter Ausbildung einer von der ersten Branche zur zweiten Branche führenden Schlaufe mit der zweiten Branche verbunden ist.

Durch die Verwendung eines zweiadrigen Versorgungskabels wird sichergestellt, daß für die elektrische Verbindung zwischen Instrument und Spannungsquelle kein Verdrillen von Anschlußkabeln auftreten kann, da die beiden die Branchen versorgenden Anschlußkabel in Form von Adern in dem zweiadrigen Versorgungskabel untergebracht und dadurch zusammengefaßt sind.

Durch die die beiden Branchen verbindende Schlaufe ist das Instrument etwa so zu handhaben, wie ein Instrument, das nur ein Anschlußkabel aufweist, welches zu einer Branche führt, da die Schlaufe zwischen den beiden Branchen bei der Handhabung des Instrumentes in keiner Weise stört.

Die Länge der Schlaufe kann beispielsweise zwischen 5 und 16 cm liegen, so daß die Schlaufe die Öffnungsbewegung der Branchen auffängt und nicht weit vom Instrument absteht.

Das Versorgungskabel kann dabei in einem Anschlußelement enden, welches an einem Anschluß der ersten Branche lösbar angeschlossen ist. Die Verbindung des gesamten Versorgungskabels erfolgt also über dieses Anschlußelement, das beispielsweise als Stecker ausgebildet sein kann, dieses Anschlußelement kann einfach von der ersten Branche gelöst werden.

Es ist dabei vorteilhaft, wenn das Verbindungskabel in das Anschlußelement einmündet und in diesem mit der zweiten Ader des Versorgungskabels verbunden ist. Dadurch wird das Ende des Verbindungskabels beim Anschließen des Anschlußelementes an den Anschluß automatisch an der ersten Branche festgelegt.

Das Verbindungskabel kann mit der zweiten Ader des Versorgungskabels dauerhaft oder lösbar verbunden sein.

Bei einer anderen Ausführungsform ist vorgesehen, daß das Verbindungskabel in den Anschluß der ersten Branche einmündet und dort lösbar mit der zweiten Ader des Versorgungskabels verbunden ist. Die Schlaufe des Verbindungskabels bleibt bei dieser Ausgestaltung dauerhaft am Instrument, das Versorgungskabel wird an den Anschluß der ersten Branche angeschlossen, dabei wird ein zweipoliger Anschluß vorgesehen, bei dem die erste Ader mit der ersten Branche und die zweite Ader mit dem Verbindungskabel verbunden werden.

Das Verbindungskabel kann dauerhaft mit der ersten Branche verbunden sein, es ist aber vorzugsweise vorgesehen, daß das Verbindungskabel lösbar mit der ersten Branche verbunden ist. Dies ist insbesondere auch zu Reinigungszwecken günstig.

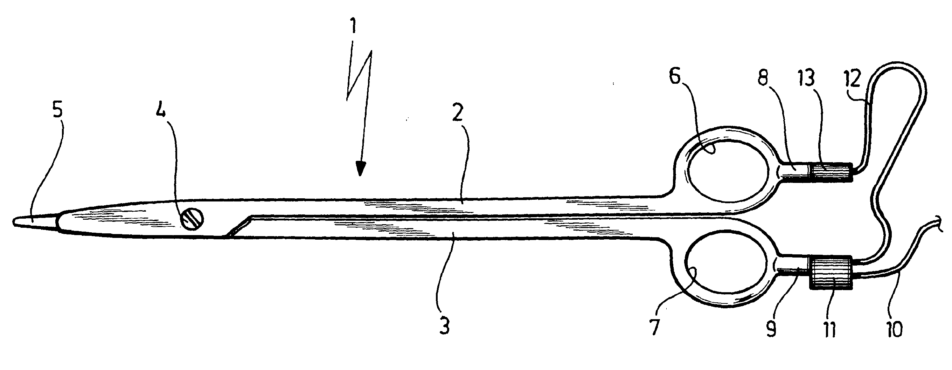

Die nachfolgende Beschreibung einer bevorzugten Ausführungsform der Erfindung dient im Zusammenhang mit der Zeichnung der näheren Erläuterung. Diese zeigt ein scherenförmigen Instrument mit einem zweiadrigen Versorgungskabel und einer schlaufenförmigen Verbindungsleitung zwischen den beiden Branchen des Instrumentes.

Das in der Zeichnung dargestellte scherenförmige Instrument 1 umfaßt zwei längliche Branchen 2, 3, die über eine Gelenkverbindung 4 relativ zueinander schwenkbar gelagert sind. An einem Ende der Branchen 2, 3 befinden sich schneidenförmige Werkzeuge 5, am gegenüberliegenden Ende tragen die Branchen 2, 3 Grifföffnungen 6 bzw. 7.

In aus der Zeichnung nicht ersichtlicher Weise sind die Branchen 2, 3 und die beiden Werkzeuge 5 elektrisch voneinander isoliert, dies kann beispielsweise durch Überzüge aus Kunststoff oder Zwischenlagen keramischer Schichten erfolgen, außerdem kann im Bereich der Gelenkverbindung 4 durch Isolierhülsen oder durch Verwendung von Lagerwellen aus isolierendem Material eine solche elektrische Isolierung erreicht werden. Dagegen sind die Branchen 2, 3 selbst mit den Werkzeugen 5 elektrisch leitend verbunden, beispielsweise können Branchen und Werkzeuge einstückig aus Metall hergestellt sein.

Bei dem in der Zeichnung dargestellten Ausführungsbeispiel tragen beide Branchen 2, 3 im Bereich ihrer Grifföffnungen 6, 7 nach hinten abstehende stiftförmige Anschlüsse 8, 9, über die die beiden Branchen 2, 3 mit den beiden Polen einer in der Zeichnung nicht dargestellten Spannungsquelle verbunden werden können, beispielsweise einem Hochfrequenzgenerator.

Die Verbindung der Anschlüsse 8, 9 mit der Spannungsquelle wird hergestellt durch ein zweiadriges Versorgungskabel 10, welches in eine Anschlußbuchse 11 einmündet, die auf den Anschluß 9 der Branche 3 aufsteckbar ist. Im Innern dieser Anschlußbuchse 11 ist eine Ader des zweiadrigen Versorgungskabels 10 so mit dieser verbunden, daß beim Aufstecken der Anschlußbuchse 11 auf den Anschluß 9 eine elektrische Verbindung zwischen dieser Ader und der Branche 3 hergestellt wird, die andere Ader des Versorgungskabels 10 ist im Innern der Anschlußbuchse 11 von dieser selbst elektrisch isoliert mit einem flexiblen Verbindungskabel 12 verbunden, welches aus der Anschlußbuchse 11 wieder austritt und in eine weitere Anschlußbuchse 13 einmündet, die derart auf den Anschluß 8 der Branche 2 aufsteckbar ist, daß das Verbindungskabel 12 elektrisch leitend mit der Branche 2 verbunden ist. Das Verbindungskabel 12 hat dabei eine Länge zwischen 5 und 16 cm und bildet eine Schlaufe aus, die die beiden Branchen 2 und 3 miteinander verbindet.

Auf diese Weise wird die Verbindung zwischen dem Instrument 1 und der Spannungsquelle über das zweiadrige Versorgungskabel 10 hergestellt, welches unmittelbar an der Branche 3 endet. Von der Branche 3 wird ein Pol der Spannungsquelle über das Verbindungskabel 12 mit der Branche 2 verbunden.

Bei dem dargestellten Ausführungsbeispiel ist das Verbindungskabel 12 mit der Branche 2 durch die Anschlußbuchse 13 lösbar verbunden, an dieser Stelle könnte auch eine dauerhafte Verbindung vorgesehen sein.

Dasselbe gilt hinsichtlich der Verbindung der Anschlußbuchse 11 mit der Branche 3, auch hier könnte die Verbindung mit dem Versorgungskabel 10 dauerhaft sein.

Bei einer anderen Ausführungsform, die in der Zeichnung nicht detailliert dargestellt ist, mündet das Verbindungskabel 12 nicht in die Anschlußbuchse 13 ein, sondern in den Anschluß 9 selbst. Die Anschlußbuchse 13 wäre dann eine zweipolige Anschlußbuchse, die die beiden Adern des Versorgungskabels 10 einerseits mit der Branche 3 und andererseits mit dem dort endenden Verbindungskabel 12 verbindet.

## Patentansprüche

1. Scheren- oder zangenförmiges chirurgisches Instrument (1) mit zwei relativ zueinander beweglich miteinander verbundenen Branchen (2, 3), die jeweils ein Werkzeug (5) tragen, wobei die Branchen (2, 3) und die Werkzeuge (5) elektrisch voneinander isoliert sind und jedes Werkzeug (5) über eine Branche (2, 3) mit jeweils einem Pol einer externen Spannungsquelle in Verbindung steht, **dadurch gekennzeichnet, daß** zu einer ersten Branche (3) ein zweiadriges, zu den Polen der externen Spannungsquelle führendes Versorgungskabel (10) führt, dessen erste Ader direkt mit dieser ersten Branche (3) verbunden ist, während dessen zweite Ader über ein Verbindungskabel (12) unter Ausbildung einer von der ersten Branche (3) zur zweiten Branche (2) führenden Schlaufe mit der zweiten Branche (2) verbunden ist.

2. Instrument nach Anspruch 1, **dadurch gekennzeichnet, daß** die Länge der Schlaufe zwischen 5 und 16 cm liegt.

3. Instrument nach einem der Ansprüche 1 oder 2, **dadurch gekennzeichnet, daß** das Versorgungskabel (10) in einem Anschlußelement (11) endet, welches an einem Anschluß (9) der ersten Branche (3) lösbar angeschlossen ist.

4. Instrument nach Anspruch 3, **dadurch gekennzeichnet, daß** das Verbindungskabel (12) in das Anschlußelement (11) einmündet und in diesem mit der zweiten Ader des Versorgungskabels (10) verbunden ist.

5. Instrument nach einem der voranstehenden Ansprüche, **dadurch gekennzeichnet, daß** das Verbindungskabel (12) mit der zweiten Ader des Versorgungskabels (10) dauerhaft verbunden ist.

6. Instrument nach einem der Ansprüche 1 bis 4, **dadurch gekennzeichnet, daß** das Verbindungskabel (12) mit der zweiten Ader des Versorgungskabels (10) lösbar verbunden ist.

7. Instrument nach Anspruch 3 und nach Anspruch 6, **dadurch gekennzeichnet, daß** das Verbindungskabel (12) in den Anschluß (9) der ersten Branche (3) einmündet und dort lösbar mit der zweiten Ader des Versorgungskabels (10) verbunden ist.

8. Instrument nach einem der voranstehenden Ansprüche, **dadurch gekennzeichnet, daß** das Verbindungskabel (12) mit der zweiten Branche (2) lösbar verbunden ist.
